# FASCICULE DE BREVET EUROPEEN

(11) **EP 2 032 083 B1**
(45) Date de publication et mention de la délivrance du brevet: **04.03.2015**
(21) Numéro de dépôt: 07789438.4
(22) Date de dépôt: 25.06.2007
(51) Int. Cl.: A61F 2/40, A61L 27/08

(54) **ASSEMBLAGE D'UNE PIECE EN PYROCARBONE ET D'UNE AUTRE PIECE**
BAUGRUPPE ZWISCHEN EINEM AUS PYROLYTISCHEM KOHLENSTOFF HERGESTELLTEN TEIL UND EINEM ANDEREN TEIL
ASSEMBLY BETWEEN A PART MADE OF PYROLYTIC CARBON AND ANOTHER PART

(30) Priorité: 29.06.2006 FR 0652712
(43) Date de publication de la demande: 11.03.2009
(73) Titulaire: BIOPROFILE, 38024 Grenoble Cedex 1 (FR)
(72) Inventeur: HASSLER, Michel, 38330 Saint-Ismier (FR); RÉAL, Cécile, 75018 Paris (FR)
(74) Mandataire: Grand, Guillaume
(86) Numéro de dépôt international: PCT/IB2007/001725
(87) Numéro de publication internationale: WO 2008/001185

(56) Documents cités:
- WO-A-2006/063363
- US-A- 5 534 033
- US-A- 5 593 445
- US-A1- 2003 208 276

## Description

La présente invention concerne l'assemblage d'une pièce en pyrocarbone et d'une autre pièce.

Le pyrocarbone est une matière qui à ce jour est utilisée avec grande satisfaction dans le domaine des prothèses médicales. L'un des intérêts du pyrocarbone est qu'il présente un module d'Young voisin de celui de l'os ainsi qu'un très bon coefficient de frottement, ce qui lui permet de travailler en frottement avec de l'os sans engendrer d'usure.

Le pyrocarbone a néanmoins pour inconvénient de mal se prêter à l'assemblage de pièces. En raison de sa fragilité en effet, il ne peut être vissé ni soudé. Il ne peut non plus être percé aussi facilement que d'autres matières telles que le métal. De ce fait, très peu de méthodes ont pu être proposées à ce jour pour accoupler une pièce en pyrocarbone à une autre pièce.

Dans le brevet US 5 458 647 est décrit un assemblage constitué d'un cylindre en pyrocarbone inséré à force dans un alésage d'une pièce métallique plus court que le cylindre. Dans cet assemblage, le pyrocarbone est maintenu en permanence sous contrainte. Un risque existe donc que le pyrocarbone se rompe dans les cas où celui-ci doit subir des contraintes supplémentaires lors du fonctionnement de l'assemblage. De plus, la zone de jonction entre la partie du cylindre en pyrocarbone engagée dans l'alésage et la partie située à l'extérieur de l'alésage constitue une zone de fragilité pouvant favoriser la rupture du pyrocarbone.

La demande de brevet EP 1 365 165 de la présente demanderesse décrit un autre type d'assemblage dans lequel une pièce en pyrocarbone définit un cône femelle dans lequel vient s'emmancher un cône mâle d'une autre pièce. Pour tenir compte de la mauvaise résistance en traction du pyrocarbone, et donc limiter les efforts en traction de la pièce femelle, une surface de la pièce femelle perpendiculaire à l'axe des cônes est en butée contre une surface correspondante de la pièce mâle. Cet assemblage maintient lui aussi en permanence le pyrocarbone sous la contrainte maximale qu'atteint le pyrocarbone lors de l'assemblage. De plus, comme dans tout accouplement conique, les cônes mâle et femelle doivent avoir une longueur de portée suffisante pour obtenir une bonne tenue du cône mâle dans le cône femelle. Un tel assemblage ne peut donc être réalisé lorsque la pièce femelle en pyrocarbone a une faible hauteur.

Le brevet US 5 593 445 décrit une prothèse comprenant une pièce annulaire en pyrocarbone encliquetée sur une tête sphérique d'une pièce mâle. La tête sphérique et la pièce annulaire en pyrocarbone sont reçues dans une pièce femelle en forme de coquille. Les pièces mâle et femelle sont faites en un alliage de titane ou en céramique. La pièce annulaire en pyrocarbone est libre de se déplacer sur la tête sphérique mais est retenue sur cette tête par l'encliquetage. Les pièces mâle et femelle sont articulées l'une à l'autre à la manière d'une rotule et peuvent toutes deux glisser sur la pièce annulaire en pyrocarbone qui est disposée entre elles. Cet assemblage de pièces mâle et femelle faites dans un matériau autre que le pyrocarbone et d'une pièce intermédiaire en pyrocarbone mobile génère des frottements importants et donc des risques d'usure.

La demande de brevet US 2003/0208276 décrit une prothèse de coude qui, selon un premier mode de réalisation, comporte une goupille d'articulation faite en un alliage de cobalt et de chrome, enfilée dans un trou d'une pièce pouvant être en pyrocarbone, trou dont le diamètre est supérieur à celui de la goupille. Selon un deuxième mode de réalisation, la pièce recevant la goupille est similaire à celle du premier mode de réalisation mais est fendue pour permettre un encliquetage de la goupille dans son trou. Le document US 2004/0030401 décrit un ensemble tel que défini dans le préambule de la revendication 1.

La présente invention vise à proposer un autre type d'assemblage d'une pièce en pyrocarbone et d'une autre pièce permettant à la pièce en pyrocarbone d'exercer une autre fonction que celles évoquées ci-dessus, à savoir une fonction de resurfaçage de l'autre pièce. Dans cette nouvelle fonction, la pièce en pyrocarbone est immobilisée sur ou dans l'autre pièce tout en étant soumise à des contraintes qui peuvent être faibles.

A cette fin, il est prévu un ensemble tel que défini à la revendication 1.

La présente invention propose aussi une prothèse, en particulier une prothèse d'épaule, comprenant l'assemblage des pièces précitées.

D'autres caractéristiques et avantages de la présente invention apparaîtront à la lecture de la description détaillée suivante faite en référence aux dessins annexés dans lesquels :
- la figure 1 est une vue en coupe d'une tête de prothèse d'épaule selon l'invention, constituée de l'assemblage d'une pièce femelle en pyrocarbone et d'une pièce mâle ;
- les figures 2 à 4 sont des demi-vues schématiques montrant le processus d'assemblage des pièces mâle et femelle de la figure 1 ;
- la figure 5 est une demi-vue schématique d'une variante de l'assemblage selon l'invention.

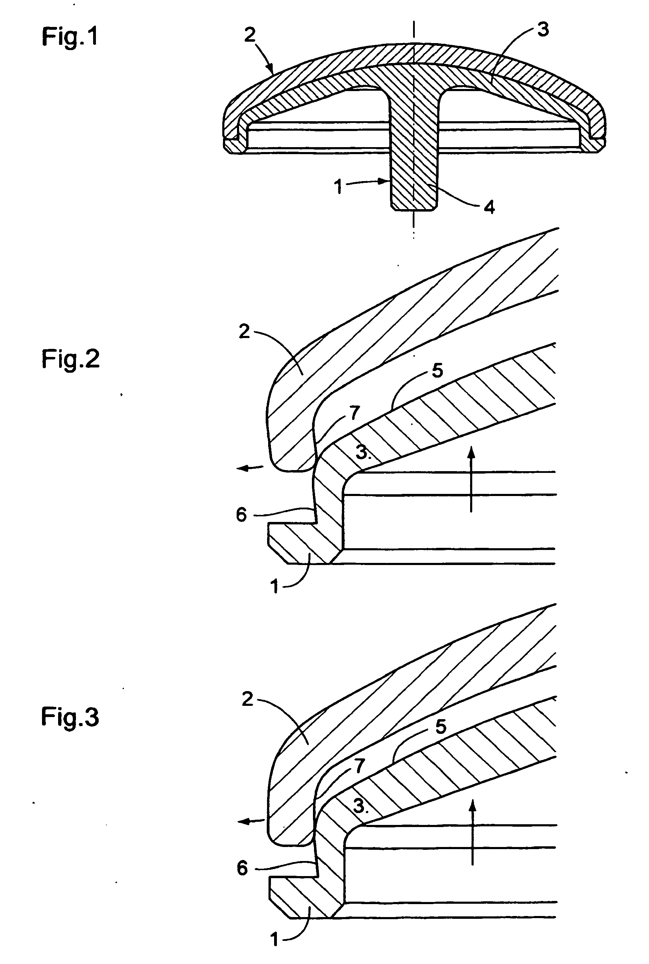

L'assemblage représenté à la figure 1 est une tête de prothèse d'épaule, comportant une pièce mâle métallique 1, faite par exemple en un alliage cobalt-chrome, accouplée à une pièce femelle en pyrocarbone 2. La pièce mâle métallique 1 définit une calotte 3 et un cône d'emmanchement 4 s'étendant depuis la surface interne de la calotte 3 le long de l'axe de la pièce 1. Le cône d'emmanchement 4 est destiné à s'accoupler avec un cône femelle d'une vis à macrofiltage (non représentée) fixée dans l'humérus. La pièce en pyrocarbone 2 est une coque de resurfaçage, qui recouvre et épouse la surface externe de la calotte métallique 3. La pièce en pyrocarbone 2 définit ainsi la surface externe, convexe, d'articulation de la prothèse, surface destinée à être en contact et en frottement avec la glène humérale.

La pièce en pyrocarbone 2 se présente typiquement sous la forme d'un substrat en graphite sur lequel est déposée une couche de pyrocarbone. La pièce 2 pourrait toutefois, en variante, être réalisée en pyrocarbone massif.

La pièce en pyrocarbone 2 est assemblée à la pièce métallique 1 par encliquetage. Un tel assemblage est rendu possible par l'élasticité du pyrocarbone, plus précisément du couple pyrocarbone/graphite, élasticité qui autorise une déformation importante de la pièce 2 sans risque de plastification. Par « encliquetage » on entend dans le cadre de la présente invention un mode d'assemblage dans lequel l'une des pièces, en l'occurrence la pièce en pyrocarbone, est déformée par l'autre pièce pendant l'assemblage et est soumise après l'assemblage à des contraintes de déformation inférieures aux contraintes de déformation maximales auxquelles elle est soumise pendant l'assemblage.

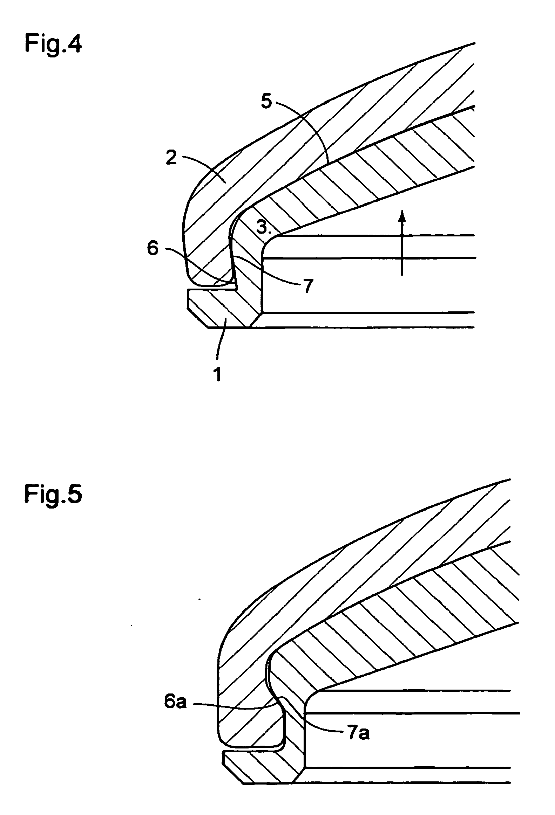

L'assemblage par encliquetage des pièces 1, 2 est illustré aux figures 2 à 4. Comme représenté sur ces figures , la calotte 3 de la pièce mâle métallique 1 comprend une surface externe supérieure convexe 5, dont le diamètre est supérieur au diamètre de l'ouverture de la pièce femelle en pyrocarbone 2, et une surface externe latérale tronconique 6 dont le diamètre va en diminuant lorsque l'on s'éloigne de la surface supérieure convexe 5 le long de l'axe de la pièce 1. Ainsi, au début de l'insertion de la pièce mâle 1 dans la pièce femelle 2, le bord de la surface supérieure 5 de la pièce mâle 1 exerce des contraintes élevées sur la pièce femelle 2 pour écarter cette dernière (figure 2). Pendant cette phase, la pièce femelle 2 peut être déformée jusqu'à un stade proche de la rupture du pyrocarbone. Le pyrocarbone n'ayant pas de domaine plastique (sa limite élastique correspond à sa limite de rupture), la déformation de la pièce femelle 2 reste à tout moment élastique. Ainsi écartée, la pièce femelle 2 glisse ensuite sur la surface latérale tronconique 6 de la pièce mâle 1 (figure 3). Au fur et à mesure de ce glissement, facilité par le faible coefficient de frottement du pyrocarbone, les contraintes reçues par la pièce femelle 2 diminuent. L'insertion de la pièce mâle 1 dans la pièce femelle 2 s'arrête lorsque le fond de la pièce femelle 2 vient buter contre la surface supérieure 5 de la pièce mâle 1. A ce moment, la pièce femelle 2 est libérée d'une partie des contraintes d'insertion et retrouve donc une forme plus proche de sa forme initiale (figure 4). Conformément à l'invention, dans cette position finale d'assemblage, la pièce femelle 2 reste soumise à des contraintes de déformation non nulles résultant de l'encliquetage. En d'autres termes, la pièce femelle 2 est maintenue dans un état légèrement écarté par la pièce mâle 1, de sorte que les pièces 1 et 2 soient solidaires par effet de serrage. La pièce femelle 2 peut ainsi exercer une fonction de resurfaçage de la pièce mâle 1. Dans cette même position finale d'assemblage, la surface latérale tronconique 6 de la pièce mâle 1 coopère avec une surface interne tronconique correspondante 7 de la pièce femelle 2 pour retenir la pièce mâle 1 dans le sens inverse de l'insertion.

L'assemblage illustré à la figure 4 est réversible. En jouant sur l'inclinaison des surfaces 6, 7, il est possible de rendre plus ou moins difficile le désassemblage des pièces mâle et femelle. La figure 5 montre une variante dans laquelle l'inclinaison des surfaces tronconiques, désignées par 6a et 7a, est accentuée, rendant plus difficile le désassemblage des pièces mâle et femelle. Dans une autre variante, l'assemblage pourrait être rendu indémontable en remplaçant les surfaces tronconiques 6, 7 par des surfaces perpendiculaires à l'axe de l'assemblage.

Les surfaces 6, 7 et 6a, 7a ne sont pas nécessairement rigoureusement tronconiques. Elles pourraient en effet avoir un profil légèrement arrondi.

Du fait que la pièce femelle 2 n'est pas destinée à être mobile par rapport à la pièce mâle 1, les pièces 1 et 2 peuvent avoir des formes très variées. En particulier, l'assemblage selon l'invention convient tout aussi bien à des pièces femelles de faible hauteur (par rapport à leur diamètre voire à leur rayon), comme la pièce 2 représentée, qu'à des pièces femelles de plus grande hauteur.

On appréciera que le mode d'assemblage selon l'invention constitue une solution pour resurfacer par exemple une pièce métallique par du pyrocarbone. Déposer du pyrocarbone sur du métal n'est en effet techniquement pas envisageable car les métaux ne résistent pas aux conditions de température dans lesquelles les dépôts de pyrocarbone sont effectués.

On notera de plus que comme, une fois assemblée à la pièce mâle, la pièce femelle en pyrocarbone est soumise à des contraintes inférieures à celles subies lors de l'insertion de la pièce mâle, la pièce femelle pourra supporter des contraintes supplémentaires pendant le fonctionnement de l'assemblage, typiquement des contraintes exercées par les os avoisinants sur la prothèse lors de mouvements du patient. Le risque de rupture du pyrocarbone pendant le fonctionnement de l'assemblage est donc réduit.

Bien que métallique dans la description ci-dessus, la pièce mâle 1 pourrait être faite dans une autre matière, par exemple en céramique. Plus généralement, la matière dans laquelle est faite la pièce mâle a de préférence un module d'Young supérieur à celui du pyrocarbone.

Enfin, la présente invention n'est pas limitée à l'assemblage d'une pièce femelle en pyrocarbone et d'une pièce mâle. Dans une variante, l'assemblage pourrait consister dans l'encliquetage d'une pièce mâle en pyrocarbone dans une pièce femelle, encliquetage obtenu au moyen d'une déformation élastique d'une partie saillante de la pièce mâle.

## Revendications

1. Ensemble comprenant une pièce en pyrocarbone (2) et une autre pièce (1), lesdites pièces (1, 2) étant conformées pour s'assembler l'une à l'autre par un encliquetage obtenu au moyen d'une déformation élastique de la pièce en pyrocarbone (2), lesdites pièces (1, 2) étant conformées pour que, lors de leur assemblage, la pièce en pyrocarbone (2) est successivement déformée élastiquement depuis une forme initiale par l'autre pièce (1), l'ensemble étant **caractérisé en ce que** la pièce en pyrocarbone (2) retrouve ensuite une forme plus proche de sa forme initiale, tout en restant soumise, en position finale d'assemblage, à des contraintes de déformation, résultant de l'encliquetage, de sorte que lesdites pièces (1, 2) sont fixement solidaires par effet de serrage.

2. Ensemble selon la revendication 1, **caractérisé en ce que** la pièce en pyrocarbone (2) est une pièce femelle et l'autre pièce (1) est une pièce mâle.

3. Ensemble selon la revendication 1 ou 2, **caractérisé en ce que** l'autre pièce (1) est en une matière ayant un module d'Young supérieur à celui du pyrocarbone.

4. Ensemble selon l'une quelconque des revendications 1 à 3, **caractérisé en ce que** l'autre pièce (1) est en métal.

5. Ensemble selon l'une quelconque des revendications 1 à 4, **caractérisé en ce que** la pièce en pyrocarbone (2) est une coque de resurfaçage de l'autre pièce (1).

6. Ensemble selon l'une quelconque des revendications 1 à 5, dans un état assemblé.

7. Prothèse comprenant un assemblage selon la revendication 6.

8. Prothèse d'épaule comprenant un assemblage selon la revendication 6.

9. Prothèse d'épaule selon la revendication 8, **caractérisée en ce que** la pièce en pyrocarbone (2) définit une surface externe d'articulation de la prothèse.

10. Prothèse d'épaule selon la revendication 8, **caractérisée en ce que** la pièce en pyrocarbone (2) définit une surface externe d'articulation avec la glène humérale.

## Patentansprüche

1. Anordnung, die ein Teil (2) aus pyrolytischem Kohlenstoff und ein anderes Teil (1) umfasst, wobei die Teile (1, 2) ausgebildet sind, durch Rasten zusammengefügt zu werden, das mittels einer elastischen Verformung des Teils (2) aus pyrolytischem Kohlenstoff erhalten wird, wobei die Teile (1, 2) bei ihrem Zusammenfügen derart ausgebildet sind, dass das Teil (2) aus pyrolytischem Kohlenstoff durch das andere Teil (1) aus einer Ursprungsform allmählich elastisch verformt wird, wobei die Anordnung **dadurch gekennzeichnet ist, dass** das Teil (2) aus pyrolytischem Kohlenstoff anschließend eine Form wiedererlangt, die näher an seiner Ursprungsform ist, wobei es in seiner Endposition des Zusammenfügens Verformungskräften unterworfen bleibt, die von dem Rasten herrühren, derart, dass die Teile (1, 2) durch eine Klemmwirkung fest verbunden sind.

2. Anordnung nach Anspruch 1, **dadurch gekennzeichnet, dass** das Teil (2) aus pyrolytischem Kohlenstoff ein Aufnahmeelement ist und das andere Teil (1) ein Einsteckelement ist.

3. Anordnung nach Anspruch 1 oder 2, **dadurch gekennzeichnet, dass** das andere Teil (1) aus einem Material besteht, das einen Elastizitätsmodul größer als der des Teils (2) aus pyrolytischem Kohlenstoff aufweist.

4. Anordnung nach einem beliebigen der Ansprüche 1 bis 3, **dadurch gekennzeichnet, dass** das andere Teil (1) ein Metall ist.

5. Anordnung nach einem beliebigen der Ansprüche 1 bis 4, **dadurch gekennzeichnet, dass** das Teil (2) aus pyrolytischem Kohlenstoff eine Schale zur Abdeckung des anderen Teils (1) ist.

6. Anordnung nach einem beliebigen der Ansprüche 1 bis 5 in einem zu einem Verbund zusammengesetzten Zustand.

7. Prothese, einen Verbund nach Anspruch 6 umfassend.

8. Schulterprothese, einen Verbund nach Anspruch 6 umfassend.

9. Schulterprothese nach Anspruch 8, **dadurch gekennzeichnet, dass** das Teil (2) aus pyrolytischem Kohlenstoff eine äußere Gelenkfläche der Prothese begrenzt.

10. Schulterprothese nach Anspruch 8, **dadurch gekennzeichnet, dass** das Teil (2) aus pyrolytischem Kohlenstoff eine äußere Gelenkfläche mit der Oberarmgelenkpfanne begrenzt.

## Claims

1. Assembly comprising a pyrocarbon part (2) and another part (1), wherein said parts (1, 2) are shaped to be fitted to one another by a locking arrangement obtained by means of an elastic deformation of the pyrocarbon part (2) and said parts (1, 2) are shaped so that during fitting the pyrocarbon part (2) is successively elastically deformed from an initial shape by the other part (1), and the assembly is **characterised in that** the pyrocarbon part (2) then returns to a shape closest to its initial shape while still being subjected to deformation stresses in the final fitting position resulting from the locking arrangement so that said parts (1, 2) are securely fitted together by clamping.

2. Assembly according to claim 1, **characterised in that** the pyrocarbon part (2) is a female part and the other part (1) is a male part.

3. Assembly according to claim 1 or 2, **characterised in that** the other part (1) is made of a material having a higher Young's modulus than that of pyrocarbon.

4. Assembly according to any one of claims 1 to 3, **characterised in that** the other part (1) is made of metal.

5. Assembly according to any one of claims 1 to 4, **characterised in that** the pyrocarbon part (2) is a resurfacing shell of the other part (1).

6. Assembly according to any one of claims 1 to 5 in a fitted state.

7. Prosthesis comprising a fitted assembly according to claim 6.

8. Shoulder prosthesis comprising a fitted assembly according to claim 6.

9. Shoulder prosthesis according to claim 8, **characterised in that** the pyrocarbon part (2) defines an external articulation surface of the prosthesis.

10. Shoulder prosthesis according to claim 8, **characterised in that** the pyrocarbon part (2) defines an external articulation surface with the glenohumeral joint.
